# EUROPEAN PATENT SPECIFICATION

(11) **EP 2 265 161 B1**
(45) Date of publication and mention of the grant of the patent: **24.08.2016**
(21) Application number: 09723538.6
(22) Date of filing: 12.03.2009
(51) Int. Cl.: A61B 10/02, A61B 5/00, A61B 17/00, A61B 90/30

(54) **BIOPSY DEVICE**
BIOPSIEVORRICHTUNG
DISPOSITIF DE BIOPSIE

(30) Priority: 18.03.2008 EP 08152902
(43) Date of publication of application: 29.12.2010
(73) Proprietor: Koninklijke Philips N.V., 5656 AE Eindhoven (NL)
(72) Inventor: BRAUN, Augustinus, L., NL-5656 AE Eindhoven (NL); HENDRIKS, Bernardus, H., W., NL-5656 AE Eindhoven (NL); VAN DER VLEUTEN, Cornelius, A., N., M., NL-5656 AE Eindhoven (NL)
(74) Representative: Kroeze, Johannes Antonius
(86) International application number: PCT/IB2009/051026
(87) International publication number: WO 2009/115952

(56) References cited:
- US-A- 4 994 060
- US-A- 5 019 040
- US-A- 5 695 482
- US-A- 5 742 429
- US-A- 6 058 323
- US-A1- 2005 203 419
- US-B1- 6 503 225

## Description

### FIELD OF THE INVENTION

The invention generally relates to a biopsy device. Particularly, the invention relates to a biopsy device comprising a shaft with fibres for optical inspection.

### BACKGROUND OF THE INVENTION

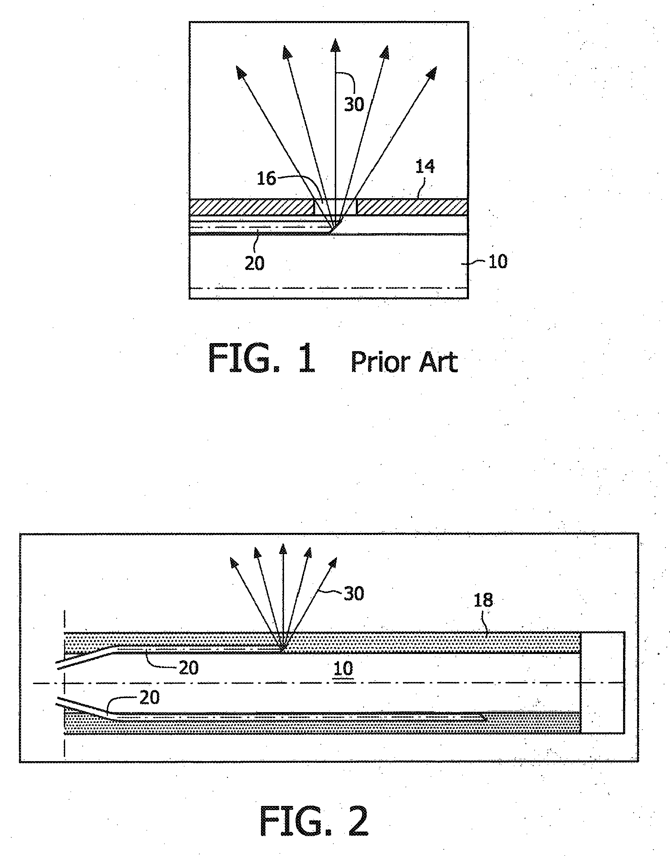

As shown in figure 1, optical fibres might be integrated in the shaft wall of a biopsy device. With these fibres optical inspection of the tissue around the device becomes possible. Light from emitting fibres is coupled out through a small hole in the outer surface of the device in a direction perpendicular to the axis of rotation of the device. This light will scatter in the tissue. The same or other fibres, also integrated in the device and in the vicinity of the emitting fibre, are used for collecting the scattered light. Both emitting and collecting fibres are mounted parallel to the axis of rotation of the device. Because the main direction of the emitted or collected light travels in a direction perpendicular to the axis of rotation of the device, the plane of the tip of the fibre shall have an inclination of 45° relative to its axis of rotation, as shown in figure 1.

However, the following problems or disadvantages exist in the prior art. In the outer surface of the biopsy needle transparent windows must be present through which the light is coupled out into the tissue or through which the light is collected from the tissue. These windows also must act as a seal to avoid penetrating of liquid and particles of the tissue into the device. Furthermore, these seals itself and the junction between fibre and seal may not obstruct the passage of the light from the fibre into the tissue and vice versa. The manufacturing and the assembly of this kind of seals is difficult, because of the small dimensions and because various fibres have to be incorporated.

US 5,019,040 discloses a catheter comprising a tubular structure made of silicone and having a channel extending through it. A connector is attached to one end of the structure, and adapted to be connected to an energy source. Energy guide fibers extend through the connector and are embedded in the tubular structure, and are adapted to be connected to the energy source.

### SUMMARY OF THE INVENTION

It is an object of the invention., to provide a biopsy device, the manufacturing of which is simplified and, thus, the costs of manufacturing are reduced.

This object is solved by the subject matter of the respective independent claims. Further exemplary embodiments are described in the dependent claims.

Generally, a biopsy device according to the invention, comprises an elongate shaft with a wall surrounding an inner space, and with a fibre having a tip and allowing the emitting and / or receiving of light, wherein the wall comprises a first material being transparent, and wherein the tip of the fibre is embedded in the first material.

According to the invention, the wall is formed completely from the first material, i.e., the complete wall of the biopsy device is transparent and the fibre or a plurality of fibres are embedded in the wall, such that the fibres will not obstruct any object introduced into the shaft. Furthermore, no edge or sharp portion of the fibres will came in contact with tissue, when a biopsy procedure is performed.

According to an embodiment of the invention, the wall of the shaft of the biopsy device further comprises a second material forming an outer tube being not transparent, wherein holes are provided in the outer tube.

The tip of the fibre comprises an end surface which is inclined relative to the axis of the elongate shaft. The advantage of the inclined surface is that the light emitted through the fibre, will be directed in a desired direction, for example, perpendicular to the axis of the shaft. By way of such an arrangement, also tissue at the side of the shaft might be inspected.

Further, a reflective layer is provided at the inclined end surface of the tip of the fibre, to improve the reflection of the emitted / received light to the desired direction.

Alternatively, an air bubble might be provided in the first material, located at the inclined end surface of the tip of the fibre, or reflective particles might be provided in front of the tip of the fibre, wherein the reflective particles might be provided in a separately formed droplet or layer.

Accordingly, the method for manufacturing a biopsy device according to the invention bases on the following steps:
1. Applying optical fibres to the surface of an inner tube. Each fibre comprises an inclined plane at the tip of the fibre, which may be covered with a reflective layer.
2. Suspend transparent liquid around these fibres, in such a way, that they are fully covered.
3. In case the inclined plane of the tip of the fibre is not covered with a reflective layer, take measures that this plane is not in contact with the transparent liquid, for example, by way of an air bubble.
4. Cure the transparent liquid to become solid.

In step 3 a mould can be used to form the outer surface of the needle. It is also possible to dip in transparent liquid.

In step 4 UV curable liquid can be used, when the transition from liquid into solid is obtained by UV illumination of the liquid, for instance Vitralit.

Also liquids can be used, consisting of two components, where the transition from liquid into solid is obtained by a chemical reaction during a certain time span, for instance Araldit.

### BRIEF DESCRIPTION OF THE DRAWINGS

In the following, the invention will be described by way of preferred embodiments with respect to the figures, wherein:
Figure 1 shows a sectional view of a shaft of a biopsy device according to the prior art, having a hole in the outer surface of the shaft.
Figure 2 shows a sectional view of a first embodiment of a biopsy device according to the invention.
Figure 3 shows three detail views of different variants of the tip of a fibre embedded in a transparent medium.
Figure 4 shows three detail views of different variants of the tip of a fibre according to a second embodiment of the invention.

### DETAILED DESCRIPTION OF EMBODIMENTS

As illustrated in figure 2, a shaft of a biopsy device according to the invention includes a construction where both emitting and receiving fibres are embedded in a transparent material forming the shaft. This material itself forms the outside surface of the biopsy device.

Accordingly, the shaft of the biopsy device comprises an inner space 10, a wall formed by a transparent material 18, at least one fibre 20 for emitting and/or receiving light. In the figures, the light emitted through a fibre, is illustrated as arrows 30.

The inner space 10 may also be a conventional needle such as a hollow metal needle for biopsy procedures, around which the transparent material 18 may be present.

For unobstructed passage of the light from the fibre 20 through the transparent material 18 into the tissue and vice versa the tip of the fibre 20 must be embedded in the transparent material.

To obtain maximum reflection of the light at the surface of the inclined plane of the fibre tip, one of the following measure shall be taken.

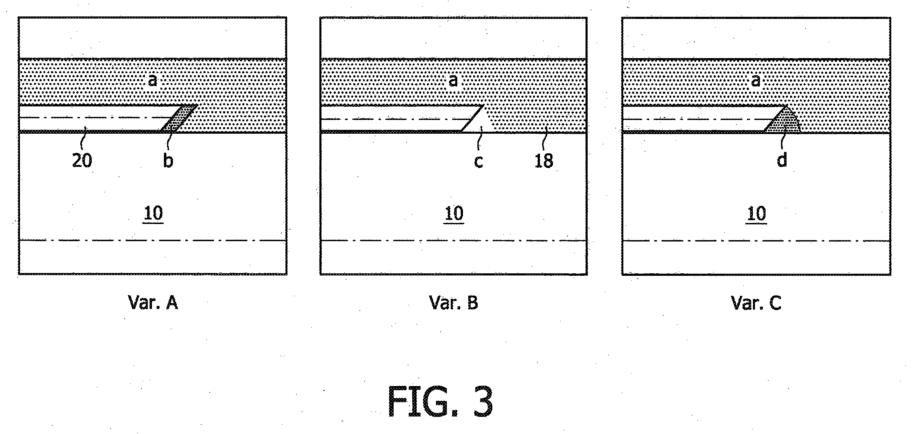

According to the variant A, shown in figure 3, a reflective layer is applied to the outer surface of the inclined plane (b) at the tip of the fibre 20.

According to the variant B, shown in figure 3, an air bubble (c) is provided in the transparent material 18 (also indicated with a) in such a way that there is only air in contact with the outer surface of the inclined plane and not the transparent curable material.

According to variant C of the first embodiment, shown in figure 3, first a curable droplet or layer, containing reflective particles (indicated with d), is provided, covering the inclined end of the fibre. In this way the inclined end of the fibre is covered with reflective particles leading to oblique outcoupling of light. Subsequently, a curable layer (a) which is fully transparent is applied to make the outer surface fully flat without sharp edges.

Since the wall of the shaft is fully transparent, it is, in principle, possible to emit the light from a tip of a fibre in any direction relative to the axis of the shaft. Thus, the light should be emitted from the tip of a respective fibre to a predefined direction, i.e. with a predefined angle, to make sure that a user of the device will know which tissue located around or inside the biopsy device, is inspected.

According to an exemplary embodiment, there is provided a plurality of fibres, each of which has a predefined inclination to the axis of the shaft, wherein the inclination might be different from one fibre to another. It is also possible that a tip of a fibre is located at the end of the shaft such that the light will be emitted in the direction of the axis of the shaft, i.e. to the front.

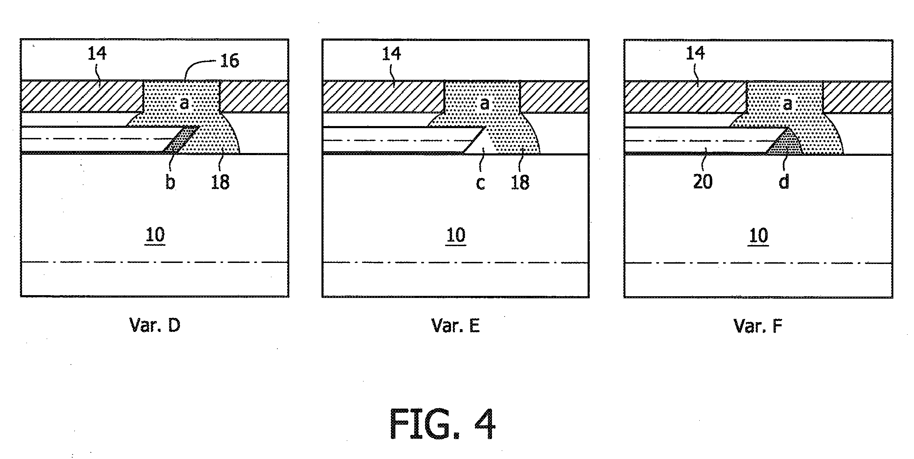

According to the second embodiment of the invention, the biopsy needle comprises an outer tube 14 made of non-transparent material, in the surface of which small holes 16 are manufactured, as shown in figure 4.

The emitting and receiving fibres are fixed relative to an inner space 10 in such a way that the tips of the fibres 20 are just below the holes 16. To avoid penetrating of liquid and particles of the tissue into the device, the holes 16 in the outer tube 14 are filled with a material 18 (also indicated as a), transparent for the emitted and collected light.

For unobstructed passage of the light from the fibre 20 through the hole 16, also the tip of the fibre must be embedded in the transparent material and the same measures might be taken as described with respect to the first embodiment of the invention.

According to variant D, as shown in figure 4, a reflective layer is applied to the outer surface of the inclined plane (b).

According to variant E, shown in figure 4, an air bubble (c) is provided in the medium (a) in such a way that there is only air in contact with the outer surface of the inclined plane and not the transparent curable layer (a).

According to variant F of the second embodiment, first a curable droplet or layer, containing reflective particles, indicated with (d), is provided, covering the inclined end of the fibre 20. In this way the inclined end of the fibre is covered with reflective particles leading to oblique outcoupling of light. A curable layer (a) which is fully transparent is then applied to make the outer surface of the shaft fully flat without sharp edges.

It should be noted, that the biopsy device according to the invention might be a biopsy needle itself, wherein the inner space is adapted to receive tissue which is intended to be analyzed. On the other hand side, the biopsy device might be a canula which will be introduced into a body to lead a biopsy needle, and through which the biopsy needle might be introduced into the body, to perform the actual biopsy.

While the invention has been illustrated and described in detail in the drawings and foregoing description, such illustration and description are to be considered illustrative or exemplary and not restrictive; the invention is not limited to the disclosed embodiments.

## Claims

1. A biopsy device comprising an elongate shaft with
- a wall surrounding an inner space (10), and
- an optical fibre (20) having a tip, the optical fibre allowing the emitting and / or receiving of light,
wherein the wall comprises a first material (18) being a transparent and solid UV cured liquid,
wherein the tip of the optical fibre (20) is embedded in the first material (18), wherein the tip of the optical fibre (20) comprises an end surface which is inclined relative to the axis of the elongate shaft,
wherein the biopsy device further comprises one out of the group consisting of (i) a reflective layer being provided at the inclined end surface of the tip of the fibre, (ii) an air bubble being provided in the first material (18) and being located at the inclined end surface of the tip of the fibre (20), and (iii) reflective particles being provided in front of the tip of the fibre (20).

2. The biopsy device of claim 1,
wherein the wall further comprises a second material,
wherein the second material is forming an outer tube (14) being not transparent,
wherein holes (16) are provided in the outer tube (14).

3. The biopsy device of claim 1,
wherein the reflective particles are provided in a separately formed droplet or layer at the inclined surface of the tip of the fibre (20).

4. A method of manufacturing a shaft of a biopsy device, the method comprising the steps of
- providing a fibre (20) having a tip and allowing the emitting and / or receiving of light, wherein an end surface at the tip of the fibre (20) is inclined relative to the axis of the elongate shaft,
performing one of the steps out of the group consisting of (i) providing a reflective layer at the inclined end surface of the tip of the fibre (20), (ii) providing an air bubble located at the inclined end surface of the tip of the fibre (20), and (iii) providing reflective particles in front of the tip of the fibre (20),
- forming a wall surrounding an inner space (10) of the shaft by suspending an UV curable liquid around the fibre so that the fibre is fully covered and curing the liquid with UV illumination to become solid,
so that the wall comprises a transparent and solid UV cured liquid,
and that the tip of the fibre (20) is embedded in the transparent and solid UV cured liquid (18).

5. The method of claim 4,
wherein the wall further comprises a second material,
wherein the second material is forming an outer tube (14) being not transparent,
wherein holes (16) are provided in the outer tube.

6. The method of claim 4, further comprising the step of
- forming a droplet or layer including reflective particles, and
- providing the droplet or layer at the inclined surface of the tip of the fibre (20).

## Patentansprüche

1. Biopsievorrichtung umfassend einen länglichen Schaft mit
- einer einen Innenraum (10) umgebenden Wand, und
- einer optischen Faser (20) mit einer Spitze, wobei die optische Faser das Aussenden und/oder Empfangen von Licht erlaubt,
wobei die Wand ein erstes Material (18) umfasst, das eine transparente und feste UV-gehärtete Flüssigkeit ist,
wobei die Spitze der optischen Faser (20) in dem ersten Material (18) eingebettet ist,
wobei die Spitze der optischen Faser (20) eine Endoberfläche umfasst, die in Bezug auf die Achse des länglichen Schafts geneigt ist,
wobei die Biopsievorrichtung weiterhin eines aus der Gruppe bestehend aus (i) einer reflektierenden Schicht, die an der geneigten Endoberfläche der Spitze der Faser vorgesehen ist, (ii) eine Luftblase, die in dem ersten Material (18) vorgesehen ist und sich an der geneigten Endoberfläche der Spitze der Faser (20) befindet, und (iii) reflektierende Partikel, die vor der Spitze der Faser (20) vorgesehen sind, umfasst.

2. Biopsievorrichtung nach Anspruch 1,
wobei die Wand weiterhin ein zweites Material umfasst,
wobei das zweite Material eine Außenröhre (14) bildet, die nicht transparent ist,
wobei Löcher (16) in der Außenröhre (14) vorgesehen sind.

3. Biopsievorrichtung nach Anspruch 1,
wobei die reflektierenden Partikel in einem separat gebildeten Tropfen oder einer separat gebildeten Schicht an der geneigten Oberfläche der Spitze der Faser (20) vorgesehen sind.

4. Verfahren zur Herstellung eines Schafts einer Biopsievorrichtung, wobei das Verfahren die folgenden Schritte umfasst:
- Vorsehen einer Faser (20), die eine Spitze hat und das Aussenden und/oder Empfangen von Licht erlaubt, wobei eine Endoberfläche der Spitze der Faser (20) in Bezug auf die Achse des länglichen Schafts geneigt ist,
- Durchführen von einem der Schritte aus der Gruppe bestehend aus (i) Vorsehen einer reflektierenden Schicht an der geneigten Endoberfläche der Spitze der Faser (20), (ii) Vorsehen einer Luftblase, die sich an der geneigten Endoberfläche der Spitze der Faser (20) befindet, und (iii) Vorsehen von reflektierenden Partikeln vor der Spitze der Faser (20),
- Bilden einer einen Innenraum (10) des Schafts umgebenden Wand durch Suspendieren einer UV-aushärtbaren Flüssigkeit um die Faser herum, so dass die Faser vollständig bedeckt ist, und Aushärten der Flüssigkeit mittels UV-Beleuchtung, damit sie fest wird,
so dass die Wand eine transparente und feste UV-gehärtete Flüssigkeit umfasst,
und dass die Spitze der Faser (20) in der transparenten und festen UVgehärteten Flüssigkeit (18) eingebettet ist.

5. Verfahren nach Anspruch 4,
wobei die Wand weiterhin in zweites Material umfasst,
wobei das zweite Material eine Außenröhre (14) bildet, die nicht transparent ist,
wobei Löcher (16) in der Außenröhre vorgesehen sind.

6. Verfahren nach Anspruch 4, weiterhin umfassend den Schritt des
- Bildens eines Tropfens oder einer Schicht umfassend reflektierende Partikel, und
- Vorsehen des Tropfens oder der Schicht an der geneigten Oberfläche der Spitze der Faser (20).

## Revendications

1. Dispositif de biopsie comprenant une tige allongée avec
- une paroi entourant un espace interne (10), et
- une fibre optique (20) ayant une pointe, la fibre optique permettant l'émission et/ou la réception de lumière,
dans lequel la paroi comprend un premier matériau (18) étant un liquide durci par UV transparent et solide,
dans lequel la pointe de la fibre optique (20) est incorporée dans le premier matériau (18),
dans lequel la pointe de la fibre optique (20) comprend une surface d'extrémité qui est inclinée par rapport à l'axe de la tige allongée,
dans lequel le dispositif de biopsie comprend en outre l'une du groupe constitué par (i) une couche réfléchissante présente au niveau de la surface d'extrémité inclinée de la pointe de la fibre, (ii) une bulle d'air présente dans le premier matériau (18) et située au niveau de la surface d'extrémité inclinée de la pointe de la fibre (20), et (iii) des particules réfléchissantes présentes en face de la pointe de la fibre (20).

2. Dispositif de biopsie selon la revendication 1,
dans lequel la paroi comprend en outre un second matériau,
dans lequel le second matériau forme un tube externe (14) qui n'est pas transparent,
dans lequel des trous (16) sont présents dans le tube externe (14).

3. Dispositif de biopsie selon la revendication 1,
dans lequel les particules réfléchissantes sont présentes sous forme d'une gouttelette ou d'une couche formée séparément au niveau de la surface inclinée de la pointe de la fibre (20).

4. Procédé de fabrication d'une tige d'un dispositif de biopsie, le procédé comprenant les étapes suivantes
- la fourniture d'une fibre (20) ayant une pointe et permettant l'émission et/ou la réception de lumière, une surface d'extrémité au niveau de la pointe de la fibre (20) étant inclinée par rapport à l'axe de la tige allongée,
la réalisation d'une des étapes du groupe constitué par (i) la fourniture d'une couche réfléchissante au niveau de la surface d'extrémité inclinée de la pointe de la fibre (20), (ii) la fourniture d'une bulle d'air située au niveau de la surface d'extrémité inclinée de la pointe de la fibre (20), et (iii) la fourniture de particules réfléchissantes en face de la pointe de la fibre (20),
- la formation d'une paroi entourant un espace interne (10) de la tige en mettant en suspension un liquide durcissable par UV autour de la fibre de telle sorte que la fibre soit complètement couverte et en durcissant le liquide au moyen d'un éclairage UV pour qu'il devienne solide,
de telle sorte que la paroi comprenne un liquide durci par UV transparent et solide,
et que la pointe de la fibre (20) soit incorporée dans le liquide durci par UV transparent et solide (18).

5. Procédé selon la revendication 4,
dans lequel la paroi comprend en outre un second matériau,
dans lequel le second matériau forme un tube externe (14) qui n'est pas transparent,
dans lequel des trous (16) sont présents dans le tube externe.

6. Procédé selon la revendication 4, comprenant en outre l'étape suivante :
- la formation d'une gouttelette ou d'une couche incluant des particules réfléchissantes, et
- la fourniture de la gouttelette ou de la couche au niveau de la surface inclinée de la pointe de la fibre (20).
